# EUROPEAN PATENT APPLICATION

(11) **EP 4 446 415 A1**
(43) Date of publication of application: **16.10.2024**
(21) Application number: 24169329.0
(22) Date of filing: 09.04.2024
(51) Int. Cl.: C12N 15/113, A61K 31/713

(54) **NUCLEIC ACID MOLECULE AND USE OF THE NUCLEIC ACID MOLECULE IN THERAPY OF DISEASES INDUCED BY EXPANSION OF TRINUCLEOTIDE CAG REPEATS**

(30) Priority: 09.04.2023 US 202363458133 P; 16.06.2023 EP 23179894; 19.03.2024 US 202418609277
(71) Applicant: Dystrogen Gene Therapies Inc., Chicago, IL 60609 (US)
(72) Inventor: OLEJNICZAK, Marta, 61-306 Poznan (PL); KOTOWSKA-ZIMMER, Anna, 62-004 Czerwonak (PL); PEWINSKA, Marianna, 60-688 Poznan (PL); LEWICKI, Paul, Tulsa, OK 74114 (US); SIEMIONOW, Krzysztof, Chicago, IL 60610 (US)
(74) Representative: Kancelaria Eupatent.pl Sp. z.o.o

(57) **Abstract**

A nucleic acid molecule composed of a duplex and loop, in which one of the duplex strands, the guide strand, comprises a sequence chosen from SEQ ID NO. 1-4, and the other strand of the duplex, the passenger strand, is at least 80% complementary to the guide strand, wherein the nucleic acid molecule forms a hairpin structure in a cell.

## Description

### TECHNICAL FIELD

This disclosure relates to RNA interference (RNAi) reagents for treatment of diseases caused by the expansion of trinucleotide CAG repeats, compositions comprising same, and use thereof to treat patients. The reagents are CAG-targeting artificial miRNA (amiRNA) with improved safety profile (compared to other CAG-targeting amiRNA).

### BACKGROUND

Huntington's disease (HD) is an example of a group of 9 hereditary disorders caused by the expansion of CAG repeat tract in unrelated genes. This mutation leads to production of toxic proteins with polyglutamine domain and progressive neurodegeneration. Currently no therapy is offered to patients and recently 3 clinical trials for HD had to be terminated prematurely due to antisense oligonucleotides (ASO) ineffectiveness or unexpected side effects. On the other hand gene therapy approaches, including RNA interference (RNAi) technology, are very promising. Vector-based RNAi tools such as short hairpin RNA (shRNA) or artificial miRNA (amiRNA) delivered to cells as viral vectors can be used for long-term inhibition of mutated gene expression. AmiRNAs are composed of siRNA insert and pri-miRNA scaffold (Kotowska-Zimmer A. et al., 2021). In animal models of polyQ disorders, amiRNAs have been shown to effectively downregulate mutant huntingtin, ataxin 1, ataxin 3 and ataxin 7 (Evers M. et al., 2018; Boudreau R. et al., 2009; Martier R. et al., 2019; Ramachandran P. et al., 2014). uniQure is currently conducting Phase I-II clinical trials of the first AAV (adeno-associated virus) gene therapy for Huntington's disease (https://clinicaltrials.gov/ct2/show/NCT04120493). However, all above mentions strategies are non-selective and target both normal and mutant transcript. Because the wild-type polyQ proteins play important functions in the cells, the safest therapeutic strategy is to target selectively the mutant variants leaving the normal protein levels unchanged. The lack of selectivity may be one of the reasons for the failure of a phase III clinical trial conducted by Roche-Ionis (NCT03761849). Allele-selectivity can be achieved using CAG repeat-targeting strategy. It takes advantage of the ability of more silencing complexes to bind to longer CAG sequences. The tandemly repeated CAG motifs in a typical normal HTT allele (~15 CAG) bind a single RNA induced silencing complex (RISC), whereas the mutant allele (> 40 CAG) accommodates two, three or more silencing complexes. The introduction of selective modification into amiRNA sequence creates an A:A mismatch with mRNA target and activates the mechanism of translation inhibition. The CAG repeats are located in the translated regions of mRNA, and the silencing complexes bound to these sequences must experience collisions with the translation machinery. Translating ribosomes easily deplete a single mismatched RISC from normal transcripts but are less efficient in displacing the more stable complexes formed by multiple RISCs on the mutant transcripts. As a result, preferential silencing of mutant huntingtin (HTT) was observed in mouse model of HD (Kotowska-Zimmer 2022). However, CAG-targeting reagents may also bind to other complementary sequences in human transcriptome and induce off-target effects, i.e. unintended silencing of other transcripts.

US9970004 and US10329566 disclose nucleic acid molecule, expression cassette, expression vector, eukaryotic host cell, induction method of RNA interference in eukaryotic host and use of nucleic acid molecule in therapy of diseases induced by expansion of trinucleotide CAG-type repeats. Despite a significant reduction in the number of off-target sites in transcriptome affected by RNAi, the disclosed solution does not sufficiently reduce the risk of side effects.

US10457940 disclose compositions and methods useful for treating Huntington's disease. In some embodiments, the disclosure provides interfering nucleic acids (e.g., artificial miRNAs) targeting the huntingtin gene (HTT) and methods of treating Huntington's disease using the same. However, the selectivity towards pathogenic sequences of this solution is not satisfactory.

### SUMMARY

Despite solutions existing up to now describing methods of RNAi reagents usage in therapy of neurodegenerative diseases, there is still a need for a allele-selective silencing of genes containing mutant CAG tracts using vector reagents targeting directly into repeat region.

The present invention provides a nucleic acid molecule, expression cassette, expression vector, eukaryotic host cell, induction method of RNA interference in eukaryotic host and use of nucleic acid molecule in therapy of diseases induced by expansion of trinucleotide CAG repeats. The solution according to the invention relates to new concept of treating hereditary human neurological diseases caused by expansion of CAG trinucleotide repeats, with the use of RNA interference technology.

To reduce the risk of off-target effects while preserving allele-selectivity, CAG-targeting amiRNA containing two or three interruptions of CUG sequence in a siRNA insert which form two or three mismatches with a target sequence, has been developed. It significantly reduced the number of potential off-target sites in human transcriptome compared to previously developed CAG-targeting amiRNA. The position of the interruption and its type are important to ensure the activity of the molecule and allele-selectivity.

The object of the invention is a nucleic acid molecule composed of a duplex and loop, in which one of the duplex strands, the guide strand, comprises a sequence chosen from SEQ ID NO. 1-4, and the other strand of the duplex, the passenger strand, is at least 80% complementary to the guide strand, wherein the nucleic acid molecule forms a hairpin structure in a cell.

Preferably, the duplex region is 19-30 base pairs long.

Preferably, the first and the second strand of the duplex are connected by a loop 4 to 15 nt long.

Preferably, the molecule comprises modified CUG-type repeats in the guide strand having 1, 2, 3, or 4 substitutions causing formation of non-canonical base pairs by interaction with targeted CAG sequences in transcripts.

Preferably, the molecule comprises 5' and 3' flanking sequences derived from natural miRNA, wherein precursor flanking sequences of natural length are shortened or not.

Preferably, the molecule comprises a loop sequence derived from a natural miRNA.

Preferably, the heterologous miRNA backbone sequence is a miR-136 backbone sequence.

Another object of the invention is expression cassette comprising a regulated or constitutive promoter, wherein the promoter is functionally connected to a sequence encoding the nucleic acid molecule specified above.

Preferably, the promoter is a polII or polIII RNA promoter.

Another object of the invention is expression vector, comprising the expression cassette specified above.

Preferably, the expression vector is a recombinant AAV (rAAV).

Preferably, the capsid protein is an AAV5 capsid protein.

Another object of the invention is eukaryotic host cell, comprising the nucleic acid molecule specified above.

Another object of the invention is cell comprising the expression cassette specified above.

Another object of the invention is eukaryotic host cell, comprising the expression vector specified above.

Another object of the invention is method for inhibiting expression of a mutant CAG expanded gene allele in a cell, the method comprising delivering of a nucleic acid molecule specified above to a eukaryotic host.

Preferably, the method comprises introducing the nucleic acid molecule into the cell by expressing the expression cassette specified above.

Preferably, the mutant CAG expanded gene allele is an allele of a gene that causes Huntington disease, spinocerebellar ataxia type 3 or dentatorubral pallidoluysian atrophy.

Preferably, the method comprises introducing the expression cassette into the cell by the expression vector specified above.

Preferably, the cell is cultured in vitro.

Another object of the invention is a method for treating a neurological disease caused by a mutant CAG expanded gene allele in a subject in need thereof, the method comprising inhibiting expression of a mutant CAG expanded gene allele in a cell of the subject by the method specified above.

Pefereably, the mutant CAG expanded gene allele is an allele of a gene that causes Huntington disease, spinocerebellar ataxia type 3 or dentatorubral pallidoluysian atrophy.

### BRIEF DESCRIPTION OF DRAWINGS

Various embodiments are herein described, by way of example only, with reference to the accompanying drawings, wherein:
FIGS. 1A-1D show examples of the sequence and structure of the amiRNAs. Interfering RNAs are formed in cells as a result of transcription and processing.
FIG. 2 shows an expression cassette.
FIGS. 3A-3B show in vitro analysis of the efficiency and allele selectivity of different amiRNAs.
FIG. 4 shows CAG repeat-targeting interfering RNA in a form of shRNA decrease mutant huntingtin level in fibroblasts from HD patient (GM04281). Western blot analysis of a protein level.
FIG. 5 shows CAG repeat-targeting interfering RNA in a form of amiRNA decrease mutant huntingtin (A), ataxin-3 (B) and atrophin-1 (C) level in fibroblasts from patients (sequentially: GM04281, GM03561 and GM013717). Western blot analysis of a protein level.
FIG. 6 shows analysis of the HTT transcript level one month post injection of amiR136-12A in YAC128 mouse model.
FIGS. 7A-7C show Western blot analysis of HTT protein levels in the brains of YAC128 mice one month post injection of AAV5 vector in two doses.
FIGS. 8A-8F show the analysis of transcript levels of genes with high level of complementarity to amiR136-12A (off-targets).

### DETAILED DESCRIPTION

The following detailed description is of the best currently contemplated modes of carrying out the invention. The description is not to be taken in a limiting sense, but is made merely for the purpose of illustrating the general principles of the invention.

This disclosure relates to RNA interference (RNAi) reagents for treatment of diseases caused by the expansion of trinucleotide CAG repeats, compositions comprising same, and use thereof to treat patients. The reagents are CAG-targeting artificial miRNA (amiRNA) with improved safety profile (compared to other CAG-targeting amiRNA)

### Methods for Treating Huntington's Disease

Methods for delivering a transgene (e.g., an inhibitory RNA, such as a amiRNA) to a subject are provided by the disclosure. The methods typically involve administering to a subject an effective amount of an isolated nucleic acid encoding an interfering RNA capable of reducing expression of huntingtin (HTT) protein, or a rAAV comprising a nucleic acid for expressing an inhibitory RNA capable of reducing expression of huntingtin protein.

In some aspects, the disclosure provides nucleic acid molecule composed of duplex and loop, in which one of the duplex strand, guide strand, contains sequence specified by a sequence chosen from SEQ ID No. 1-4, and second strand from the duplex, passenger strand, is complementary in at least 80%, wherein nucleic acid molecule forms hairpin/ structure in the cell. In some embodiments, molecule contains in the guide strand modified CUG-type repeats containing 1, 2, 3, or 4 substitutions causing formation of non-canonical base pairs by interaction with targeted CAG sequence in transcripts. In some embodiments, duplex region is 19-30 base pairs long. In some embodiments, first and second strand of the duplex is connected by a loop 4 to 15 nt long. In some embodiments, molecule contains flanking sequences 5' and 3' derived from natural miRNA, wherein precursor flanking sequences of natural length are shortened or not. In some embodiments, molecule contains loop sequence derived from natural miRNA.

As used herein, "Huntington's disease", or "HD", refers to a neurodegenerative disease characterized by progressively worsening movement, cognitive and behavioral changes caused by a tri-nucleotide repeat expansion (e.g., CAG, which is translated into a poly-Glutamine, or PolyQ, tract) in the HTT gene that results in production of pathogenic mutant huntingtin protein (HTT, or mHTT). In some embodiments, mutant huntingtin protein accelerates the rate of neuronal cell death in certain regions of the brain. Generally, the severity of HD is correlated to the size of the tri-nucleotide repeat expansion in a subject. For example, a subject having a CAG repeat region comprising between 36 and 39 repeats is characterized as having "reduced penetrance" HD, whereas a subject having greater than 40 repeats is characterized as having "full penetrance" HD. The number of CAG repeats in a HTT gene allele of a subject can be determined by any suitable modality known in the art. For example, nucleic acids (e.g., DNA) can be isolated from a biological sample (e.g., blood) of a subject and the number of CAG repeats of a HTT allele can be determined by a hybridization-based method, such as PCR or nucleic acid sequencing (e.g., Illumina sequencing, Sanger sequencing, SMRT sequencing, etc.).

An "effective amount" of a substance is an amount sufficient to produce a desired effect. In some embodiments, an effective amount of an isolated nucleic acid is an amount sufficient to transfect (or infect in the context of rAAV mediated delivery) a sufficient number of target cells of a target tissue of a subject. In some embodiments, a target tissue is central nervous system (CNS) tissue (e.g., brain tissue, spinal cord tissue, cerebrospinal fluid (CSF), etc.). In some embodiments, an effective amount of an isolated nucleic acid (e.g., which may be delivered via an rAAV) may be an amount sufficient to have a therapeutic benefit in a subject, e.g., to reduce the expression of a pathogenic gene or protein (e.g., HTT), to extend the lifespan of a subject, to improve in the subject one or more symptoms of disease (e.g., a symptom of Huntington's disease), etc. The effective amount will depend on a variety of factors such as, for example, the species, age, weight, health of the subject, and the tissue to be targeted, and may thus vary among subject and tissue.

### Isolated Nucleic Acids

In some aspects, the disclosure provides nucleic acid molecule that are useful for reducing (e.g., inhibiting) expression of human huntingtin (HTT). A "nucleic acid" sequence refers to a DNA or RNA sequence. In some embodiments, proteins and nucleic acids of the disclosure are isolated. As used herein, the term "isolated" means artificially produced or isolated from its natural environment. As used herein with respect to nucleic acids, the term "isolated" means: (i) amplified in vitro by, for example, polymerase chain reaction (PCR); (ii) recombinantly produced by cloning; (iii) purified, as by cleavage and gel separation; or (iv) synthesized by, for example, chemical synthesis. An isolated nucleic acid is one which is readily manipulable by recombinant DNA techniques well known in the art. Thus, a nucleotide sequence contained in a vector in which 5' and 3' restriction sites are known or for which polymerase chain reaction (PCR) primer sequences have been disclosed is considered isolated but a nucleic acid sequence existing in its native state in its natural host is not. An isolated nucleic acid may be substantially purified, but need not be. For example, a nucleic acid that is isolated within a cloning or expression vector is not pure in that it may comprise only a tiny percentage of the material in the cell in which it resides. Such a nucleic acid is isolated, however, as the term is used herein because it is readily manipulable by standard techniques known to those of ordinary skill in the art. As used herein with respect to proteins or peptides, the term "isolated" refers to a protein or peptide that has been isolated from its natural environment or artificially produced (e.g., by chemical synthesis, by recombinant DNA technology, etc.).

The isolated nucleic acids of the invention may be recombinant adeno-associated virus (AAV) vectors (rAAV vectors). The isolated nucleic acid (e.g., the recombinant AAV vector) may be packaged into a capsid protein and administered to a subject and/or delivered to a selected target cell. "Recombinant AAV (rAAV) vectors" are typically composed of, at a minimum, a transgene and its regulatory sequences, and 5' and 3' AAV inverted terminal repeats (ITRs). The transgene may comprise, as disclosed elsewhere herein, one or more regions that encode one or more inhibitory RNAs (e.g., amiRNAs) comprising a nucleic acid that targets an endogenous mRNA of a subject. The transgene may also comprise a region encoding, for example, a protein and/or an expression control sequence (e.g., a poly-A tail).

The vector also includes conventional control elements which are operably linked with elements of the transgene in a manner that permits its transcription, translation and/or expression in a cell transfected with the vector or infected with the virus produced by the invention. As used herein, "operably linked" sequences include both expression control sequences that are contiguous with the gene of interest and expression control sequences that act in trans or at a distance to control the gene of interest. Expression control sequences include appropriate transcription initiation, termination, promoter and enhancer sequences; efficient RNA processing signals such as splicing and polyadenylation (polyA) signals; sequences that stabilize cytoplasmic mRNA; sequences that enhance translation efficiency (i.e., Kozak consensus sequence); sequences that enhance protein stability; and when desired, sequences that enhance secretion of the encoded product. A number of expression control sequences, including promoters which are native, constitutive, inducible and/or tissue-specific, are known in the art and may be utilized.

As used herein, a nucleic acid sequence (e.g., coding sequence) and regulatory sequences are said to be operably linked when they are covalently linked in such a way as to place the expression or transcription of the nucleic acid sequence under the influence or control of the regulatory sequences. If it is desired that the nucleic acid sequences be translated into a functional protein, two DNA sequences are said to be operably linked if induction of a promoter in the 5' regulatory sequences results in the transcription of the coding sequence and if the nature of the linkage between the two DNA sequences does not (i) result in the introduction of a frame-shift mutation, (ii) interfere with the ability of the promoter region to direct the transcription of the coding sequences, or (iii) interfere with the ability of the corresponding RNA transcript to be translated into a protein. Thus, a promoter region would be operably linked to a nucleic acid sequence if the promoter region were capable of effecting transcription of that DNA sequence such that the resulting transcript might be translated into the desired protein or polypeptide. Similarly two or more coding regions are operably linked when they are linked in such a way that their transcription from a common promoter results in the expression of two or more proteins having been translated in frame. In some embodiments, operably linked coding sequences yield a fusion protein. In some embodiments, operably linked coding sequences yield a functional RNA (e.g., miRNA).

In some aspects, the disclosure provides an isolated nucleic acid comprising a transgene, wherein the transgene comprises a nucleic acid sequence microRNAs (e.g., miRNAs). A"microRNA" or"miRNA" is a small non-coding RNAmolecule capable of mediating transcriptional or post-translational gene silencing. Typically, miRNA is transcribed as a hairpin or stem-loop (e.g., having a self-complementarity, single-stranded backbone) duplex structure, referred to as a primary miRNA (pri-miRNA), which is enzymatically processed (e.g., by Drosha, DGCR8, Pasha, etc.) into a pre-miRNA. The length of a pri-miRNA can vary.
Generally, pre-miRNA is exported into the cytoplasm, and enzymatically processed by Dicer to first produce an imperfect miRNA/miRNA*duplex and then a single-stranded mature miRNA molecule, which is subsequently loaded into the RNA-induced silencing complex (RISC). Typically, a mature miRNA molecule ranges in size from about 19 to about 30 base pairs in length.

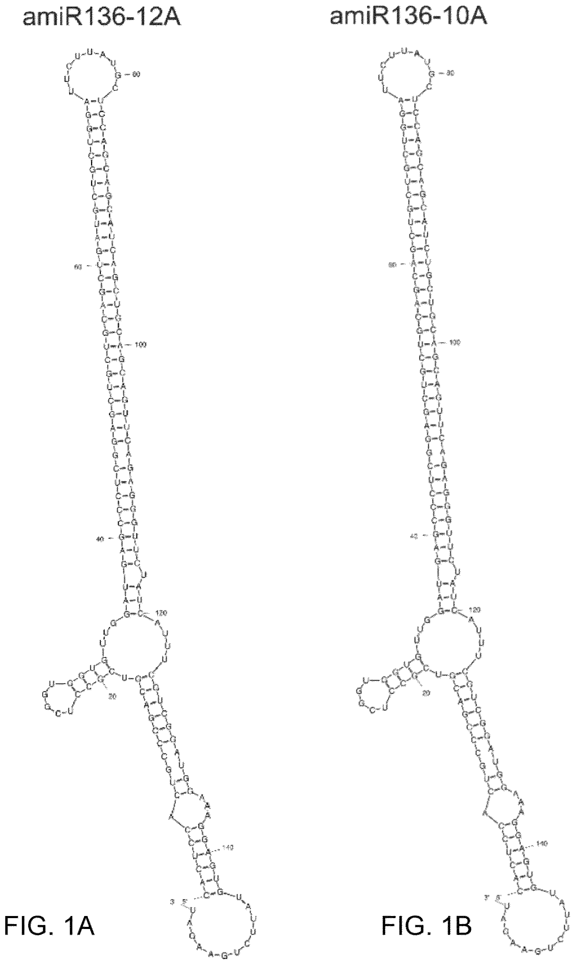

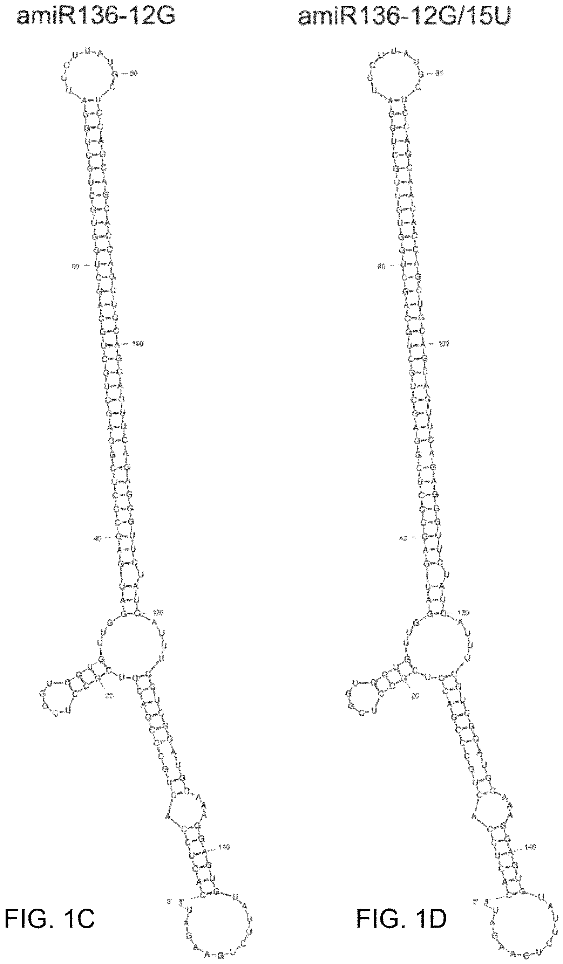

In some aspects, the disclosure provides isolated nucleic acids and vectors (e.g., rAAV vectors) that encode one or more artificial miRNAs. As used herein "artificial miRNA" or "amiRNA" refers to an endogenous pri-miRNAor pre-miRNA(e.g., a miRNA backbone, which is a precursor miRNA capable of producing a functional mature miRNA), in which the miRNA and miRNA* (e.g., passenger strand of the miRNA duplex) sequences have been replaced with corresponding siRNA/siRNA* sequences that direct highly efficient RNA silencing of the targeted gene, for example as described by Eamens et al. (2014), Methods Mol. Biol. 1062:211-224. For example, in some embodiments an artificial miRNA comprises a miR-136 pri-miRNA backbone into which a sequence encoding a mature HTT-specific siRNA (e.g., any one of SEQ ID NOs: 1-4) has been inserted in place of the endogenous miR-136 mature miRNA-encoding sequence (FIG. 1A-1D). Interfering RNAs are formed in cells as a result of transcription and processing. In some embodiments, miRNA (e.g., an artificial miRNA) as described by the disclosure comprises a miR-136 backbone sequence.

A "promoter" refers to a DNA sequence recognized by the synthetic machinery of the cell, or introduced synthetic machinery, required to initiate the specific transcription of a gene. The phrases "operatively positioned," "under control" or "under transcriptional control" means that the promoter is in the correct location and orientation in relation to the nucleic acid to control RNA polymerase initiation and expression of the gene.

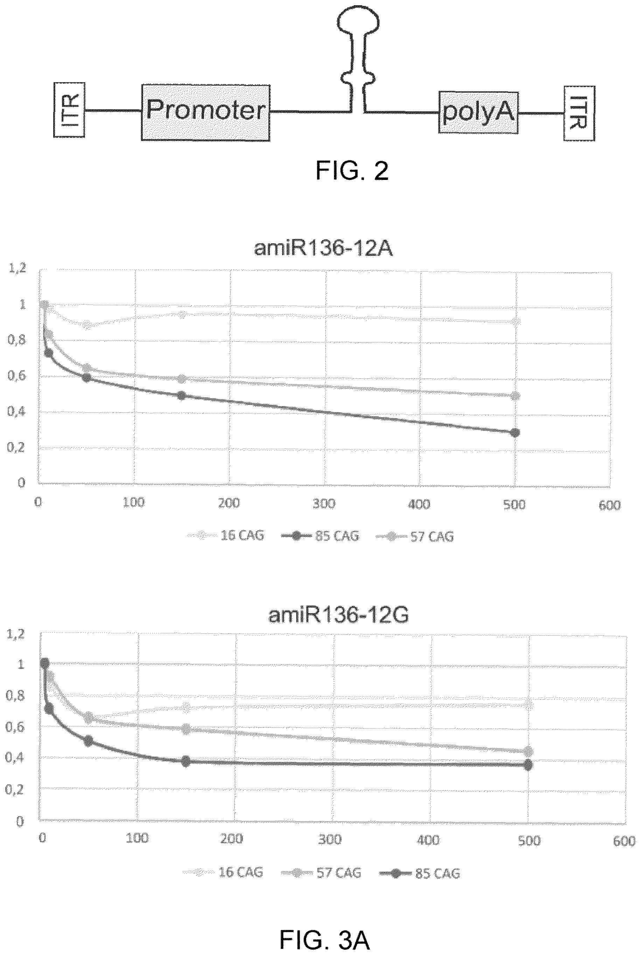

An "expression cassette" refers to a distinct component of a vector DNA consisting of a gene and a regulatory sequence to be expressed by a transfected cell. An expression cassette comprises three components: a promoter sequence, amiRNA sequence, and a transcription termination region that, in eukaryotes, usually contains a polyadenylation site. An example of the expression cassette is shown in FIG. 2.

Without wishing to be bound by any particular theory, allele-specific silencing of mutant huntingtin (HTT) may provide an improved safety profile in a subject compared to non-allele specific silencing (e.g., silencing of both wild-type and mutant HTT alleles) because wild-type HTT expression and function is preserved in the cells. Allele-selectivity can be achieved using CAG repeat-targeting strategy. It takes advantage of the ability of more silencing complexes to bind to longer CAG sequences. The introduction of selective modification into amiRNA sequence creates two or three mismatches with mRNA target and activates the mechanism of translation inhibition. As a result, preferential silencing of mutant huntingtin (HTT) was observed.

### Recombinant Adeno-Associated Viruses (rAAVs)

In some aspects, the disclosure provides isolated AAVs. As used herein with respect to AAVs, the term "isolated" refers to an AAV that has been artificially produced or obtained. Isolated AAVs may be produced using recombinant methods. Such AAVs are referred to herein as "recombinant AAVs". Recombinant AAVs (rAAVs) preferably have tissue-specific targeting capabilities, such that a nuclease and/or transgene of the rAAV will be delivered specifically to one or more predetermined tissue(s). The AAV capsid is an important element in determining these tissue-specific targeting capabilities. Thus, an rAAV having a capsid appropriate for the tissue being targeted can be selected.

Methods for obtaining recombinant AAVs having a desired capsid protein are well known in the art. Typically the methods involve culturing a host cell which contains a nucleic acid sequence encoding an AAV capsid protein; a functional rep gene; a recombinant AAV vector composed of, AAV inverted terminal repeats (ITRs) and a transgene; and sufficient helper functions to permit packaging of the recombinant AAV vector into the AAV capsid proteins. In some embodiments, capsid proteins are structural proteins encoded by the cap gene of an AAV. AAVs comprise three capsid proteins, virion proteins 1 to 3 (named VP1, VP2 and VP3), all of which are transcribed from a single cap gene via alternative splicing. In some embodiments, the molecular weights of VP1, VP2 and VP3 are respectively about 87 kDa, about 72 kDa and about 62 kDa. In some embodiments, upon translation, capsid proteins form a spherical 60-mer protein shell around the viral genome. In some embodiments, the functions of the capsid proteins are to protect the viral genome, deliver the genome and interact with the host. In some aspects, capsid proteins deliver the viral genome to a host in a tissue specific manner.

In some embodiments, an AAV capsid protein is of an AAV serotype selected from the group consisting of AAV2, AAV3, AAV4, AAV5, AAV6, AAV8, AAVrh8, AAV9, and AAV10. In some embodiments, an AAV capsid protein is of a serotype derived from a non-human primate, for example AAVrh8 serotype. In some embodiments, an AAV capsid protein is of an AAV9 serotype.

### Modes of Administration

The rAAVs of the disclosure may be delivered to a subject in compositions according to any appropriate methods known in the art. For example, an rAAV, preferably suspended in a physiologically compatible carrier (i.e., in a composition), may be administered to a subject, i.e. host animal, such as a human, mouse, rat, cat, dog, sheep, rabbit, horse, cow, goat, pig, guinea pig, hamster, chicken, turkey, or a non-human primate (e.g., Macaque). In some embodiments a host animal does not include a human.

Delivery of the rAAVs to a mammalian subject may be by, for example, intramuscular injection or by administration into the bloodstream of the mammalian subject. Administration into the bloodstream may be done by injection into a vein, an artery, or any other vascular conduit. In some embodiments, the rAAVs are administered into the bloodstream by way of isolated limb perfusion, a technique well known in the surgical arts, the method essentially enabling the artisan to isolate a limb from the systemic circulation prior to administration of the rAAV virions. Moreover, in certain instances, it may be desirable to deliver the virions to the CNS of a subject. By "CNS" is meant all cells and tissue of the brain and spinal cord of a vertebrate. Thus, the term includes, but is not limited to, neuronal cells, glial cells, astrocytes, cerebrospinal fluid (CSF), interstitial spaces, bone, cartilage and the like. Recombinant AAVs may be delivered directly to the CNS or brain by injection into, e.g., the ventricular region, as well as to the striatum (e.g., the caudate nucleus or putamen of the striatum), spinal cord and neuromuscular junction, or cerebellar lobule, with a needle, catheter or related device, using neurosurgical techniques known in the art, such as by stereotactic injection. In some embodiments, rAAV as described in the disclosure are administered by intravenous injection. In some embodiments, the rAAV are administered by intracerebral injection. In some embodiments, the rAAV are administered by intrathecal injection. In some embodiments, the rAAV are administered by intrastriatal injection. In some embodiments, the rAAV are delivered by intracranial injection. In some embodiments, the rAAV are delivered by cisterna magna injection. In some embodiments, the rAAV are delivered by cerebral lateral ventricle injection. Aspects of the instant disclosure relate to compositions comprising a recombinant AAV comprising a capsid protein and a nucleic acid encoding a transgene, wherein the transgene comprises a nucleic acid sequence encoding amiRNAs. In some embodiments, amiRNA comprises a sequence set forth in any one of SEQ ID NOs: 1-4. In some embodiments, a composition further comprises a pharmaceutically acceptable carrier.

Suitable carriers may be readily selected by one of skill in the art in view of the indication for which the rAAV is directed. For example, one suitable carrier includes saline, which may be formulated with a variety of buffering solutions (e.g., phosphate buffered saline). Other exemplary carriers include sterile saline, lactose, sucrose, calcium phosphate, gelatin, dextran, agar, pectin, peanut oil, sesame oil, and water. The selection of the carrier is not a limitation of the present disclosure.

Optionally, the compositions of the disclosure may contain, in addition to the rAAV and carrier(s), other conventional pharmaceutical ingredients, such as preservatives, or chemical stabilizers. Suitable exemplary preservatives include chlorobutanol, potassium sorbate, sorbic acid, sulfur dioxide, propyl gallate, the parabens, ethyl vanillin, glycerin, phenol, and parachlorophenol. Suitable chemical stabilizers include gelatin and albumin.

The rAAVs are administered in sufficient amounts to transfect the cells of a desired tissue and to provide sufficient levels of gene transfer and expression without undue adverse effects. Conventional and pharmaceutically acceptable routes of administration include, but are not limited to, direct delivery to the selected organ (e.g., direct delivery to the striatum ), oral, inhalation (including intranasal and intratracheal delivery), intraocular, intravenous, intramuscular, subcutaneous, intradermal, intratumoral, and other parental routes of administration. Routes of administration may be combined, if desired.

Formulation of pharmaceutically-acceptable excipients and carrier solutions is well-known to those of skill in the art, as is the development of suitable dosing and treatment regimens for using the particular compositions described herein in a variety of treatment regimens.

Typically, these formulations may contain at least about 0.1% of the active compound or more, although the percentage of the active ingredient(s) may, of course, be varied and may conveniently be between about 1 or 2% and about 70% or 80% or more of the weight or volume of the total formulation. Naturally, the amount of active compound in each therapeutically-useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

### Kits and Related Compositions

The agents described herein may, in some embodiments, be assembled into pharmaceutical or diagnostic or research kits to facilitate their use in therapeutic, diagnostic or research applications. A kit may include one or more containers housing the components of the disclosure and instructions for use. Specifically, such kits may include one or more agents described herein, along with instructions describing the intended application and the proper use of these agents. In certain embodiments agents in a kit may be in a pharmaceutical formulation and dosage suitable for a particular application and for a method of administration of the agents.

Kits for research purposes may contain the components in appropriate concentrations or quantities for running various experiments.

The kit may contain any one or more of the components described herein in one or more containers. As an example, in one embodiment, the kit may include instructions for mixing one or more components of the kit and/or isolating and mixing a sample and applying to a subject. The kit may include a container housing agents described herein. The agents may be in the form of a liquid, gel or solid (powder). The agents may be prepared sterilely, packaged in syringe and shipped refrigerated. Alternatively it may be housed in a vial or other container for storage. A second container may have other agents prepared sterilely. Alternatively the kit may include the active agents premixed and shipped in a syringe, vial, tube, or other container.

Exemplary embodiments of the invention will be described in more detail by the following examples. These embodiments are exemplary of the invention, which one skilled in the art will recognize is not limited to the exemplary embodiments.

### EXAMPLES

### Example 1: Materials and Methods

### Cell Cultures and Transfection

Fibroblasts derived from the patients with HD (GM04281-17/68 CAG repeats in HTT gene),SCA3 (GM06153-18/69 CAG repeats in ATXN3 gene) and DRPLA (GM013717 - 16/68 CAG repeats in ATN1 gene) were obtained from Coriell Cell Repositories (Camden, N.J., USA). Cells were cultured in MEM medium (Lonza; Basel, Switzerland) enriched with 8% FBS (Sigma-Aldrich; St. Louis, USA), antibiotics: penicillin, streptomycin, amphotericin B (Sigma-Aldrich) and amino acids (Cat. no. M7145, Sigma-Aldrich). For luciferase assays, HEK293T cells were cultured in 24-well plates in DMEM supplemented with 10% FBS. The next day, the cells were cotransfected with two types of plasmids: constructs containing exon 1 of the HTT gene with defined numbers of CAG repeats (16, 57 and 85) with Renilla and firefly luciferase sequences, and constructs containing amiRNAs, using Lipofectamine 2000 (Invitrogen, Thermo Fisher Scientific, Carlsbad, CA, USA). Cells were cotransfected with 50 ng of the HTT target reporter plasmid, and 5, 10, 50, 150, or 500 ng of the amiRNA construct. Forty-eight hours after transfection, cells were harvested and lysed using Passive Lysis Buffer (Promega, Madison, WI, USA). The bioluminescence assay was performed using a Dual-Luciferase^{®} Reporter Assay System (Promega) and Victor x4 Multilabel Plate Reader (Perkin Elmer, Waltham, MA, USA) according to the manufacturer's instructions. Empty plasmid was used as the negative control, and the fluorescence intensity of firefly luciferase was normalized to the fluorescence intensity of Renilla luciferase.

### Mouse Housing, Injections, Maintenance and Tissue Extraction

All experiments were performed on YAC128 transgenic (FVB-Tg(YAC128)53Hay/J) and wild-type (FVB/NJ) mice maintained on the FVB/NJ strain background. Mice were acquired from The Jackson Laboratory and bred in the animal facility of Center for Advanced Technologies Adam Mickiewicz University in Poznan (CAT AMU) where the experiments were conducted. All experiments were approved by the Local Ethical Committee for Animal Experiments. Genotypes were verified by PCR of DNA extracted from tail snips. Animals were housed under specific pathogen-free (SPF) conditions in individually ventilated cages (IVCs) with access to water and food ad libitum. Mice were injected with 3 µl of AAV5 vectors directly into the striatum of both hemispheres at specific coordinates (AP + 0.7 mm, ML ± 1.7 mm, and DV -3.5 mm from the bregma) using a Hamilton gauge syringe over a 10-minute period (0.3 µl/min). All surgeries were performed under inhaled isoflurane anesthesia, and mice were placed on a heating pad to prevent hypothermia. The wound was covered with antibiotics to prevent infection. After surgery, mice were injected subcutaneously with a nonsteroidal anti-inflammatory drug (meloxicam) and transferred to preheated cages for recovery. The health of mice was monitored for at least 2 hours postsurgery and afterward on a daily basis. One month post injection mice were sacrificed and tissue extracted for RNA, DNA and protein analysis.

### Design and Selection of Huntingtin Targeting Artificial miRNAs

Previously described CAG-targeting amiR136-A2 molecule contains 10 off-target sequences with 100% complementarity in human genome. Therefore, we used the NCBI BLAST algorithm (Ref seq - RNA sequences; Homo Sapiens; Megablast; match/mismatch scores: 4/-5; gap costs: Existence 12, Extension 8) to match the A2 sequence to the human genome and identify nucleotide positions in the A2 sequence whose change would result in a lack of complete complementarity of the molecule to the transcriptome. Any nucleotide change at position 8, 9, 10, 12 or 15 in the A2 molecule results in no perfect match to the human transcriptome and significant reduction of other potential binding sites with no perfect complementarity. Because purine-purine mismatches have the highest discriminative potential, we introduced A and G nucleotides in selected positions of the A2 molecule. In the first step we designed 8A, 9A, 10A, 12A and 12G in a form of shRNA and tested their efficacy and allele-selectivity in vitro (HTT, luciferase tests). Based on these analyses, the most efficient and selective shRNAs were selected, their activity in HD fibroblasts was confirmed, and their corresponding amiRNAs (amiR136-10A, amiR136-12A, amiR-12G and amiR-12G/15U) were designed.

### Western Blotting

Western blot analysis for HTT protein Briefly, 30 µg of total protein was separated on a Tris-acetate sodium dodecyl sulfate (SDS)-polyacrylamide gel (1.5 cm, 4% stacking gel/4.5 cm, 5% resolving gel, acrylamide:bis-acrylamide ratio of 49:1) in XT Tricine buffer (Bio-Rad, Hercules, CA, USA) at 135 V in an ice-water bath. For proteins isolated from mouse brains, NuPAGE Tris-Acetate 3%-8% Protein Gel (Thermo Fisher Scientific) in NuPAGE Tris-Acetate SDS Running Buffer (Thermo Fisher Scientific) were used. After electrophoresis, the proteins were transferred overnight to a nitrocellulose membrane (Sigma-Aldrich) by the wet transfer method. The primary and secondary antibodies were used in PBS/0.1% Tween-20 buffer containing 5% nonfat milk. Immunoreactions were detected using Western Bright Quantum HRP Substrate (Advansta, Menlo Park, CA, USA). Protein bands were scanned directly from the membrane using a camera, and band densities were quantified using a Gel-Pro Analyzer (Media Cybernetics). Plectin or calnexin was used as the reference protein.

### RNA Isolation and RT-qPCR

Total RNA was isolated using TRIzol Reagent (Thermo Fisher Scientific) and Phenol equilibrated, stabilized chloroform: isoamyl alcohol 25:24:1 (PanReac Applichem, Barcelona, Spain). A DeNovix Nanodrop Spectrophotometer was used to measure the RNA concentration. A total of 500 ng of total RNA was transcribed to cDNA using SuperScript^{™} III Reverse Transcriptase (Invitrogen) at 55°C. RT-qPCR was performed in a the CFX Connect Real-Time PCR Detection System (Bio-Rad, Hercules, CA, USA) using SsoAdvanced Universal SYBR Green Supermix (Bio-Rad) with β-actin as the reference gene under the following thermal cycling conditions: denaturation at 95°C for 30 s followed by 40 cycles of denaturation at 95°C for 15 s and annealing at 60°C for 30 s. Gene expression levels were normalized to those in vehicle-treated mice.

### Plasmids and Virus Vectors

For experiments performed in cell cultures the amiRNA expression cassettes were generated from DNA oligonucleotides. Pairs of oligonucleotides were annealed and ligated into the pCDH-CMV-MCS-EF1-Puro (System Biosciences, Palo Alto, CA, USA) expression plasmid and verified through sequencing. For lentivirus production, the plasmids were cotransfected with the packaging plasmids pPACKH1-GAG, pPACKH1-REV, and pVSVG (System Biosciences) into HEK293TN cells. The medium was collected on Days 2 and 3, and the viral supernatants were passed through 0.45-µm filters and concentrated using PEGit Virus Precipitation Solution (System Biosciences). The lentiviral vectors were resuspended in Opti-MEM (GIBCO, Invitrogen, Carlsbad, CA, USA), and the virus titers (TU/mL) were determined through flow cytometry (Accuri C6, BD Biosciences, San Jose, CA, USA) based on copGFP expression. Transduction of fibroblasts was performed at MOI of 10 in the presence of polybrene (4 µg/mL). Total protein was harvested 7 days post transduction. The luciferase (Luc) construct was used as a negative control.

For in vivo experiments, the amiRNA constructs were used for the production of the AAV5 vectors. The amiRNAs were expressed under the control of the CAG Pol II promoter. We used empty constructs as negative controls for silencing (vehicle control). AAV5 vectors were produced in the HEK293 cell system by Virovek (Houston, TX, USA).

### Example 2: Cell lines experiments

### Allele selectivity of different amiRNAs

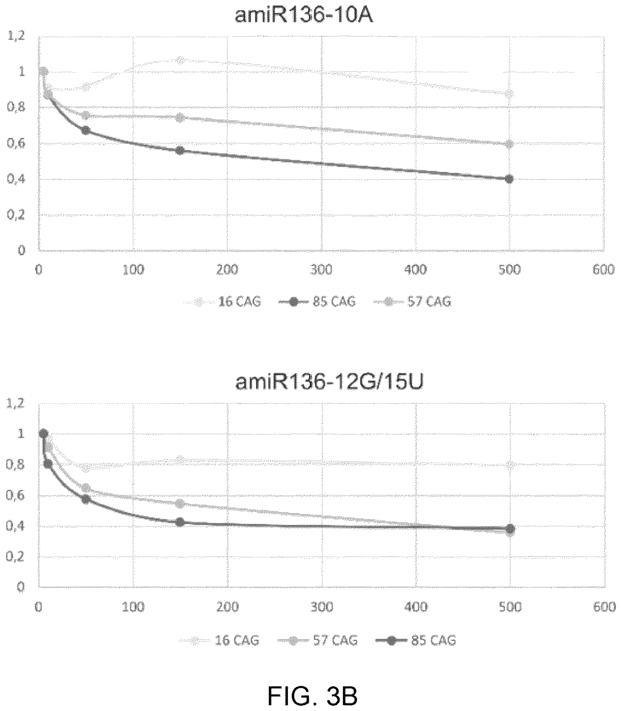

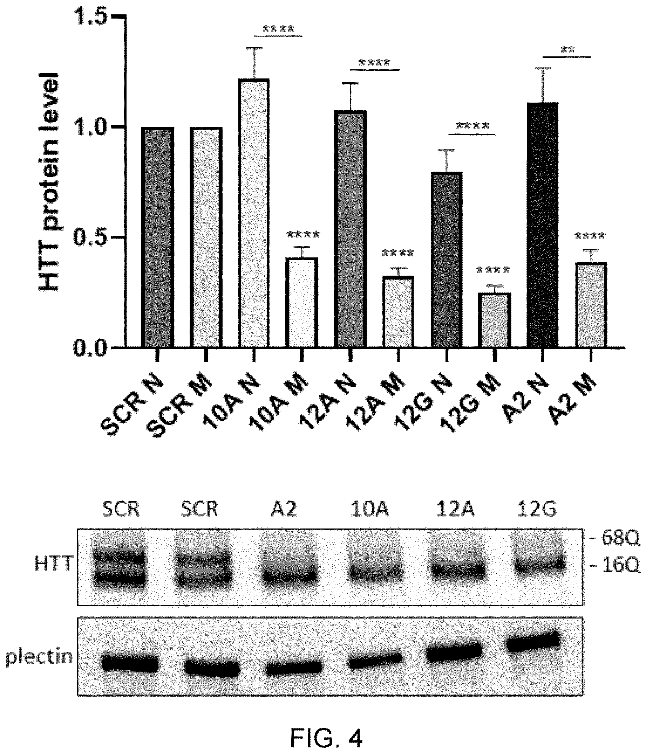

Allele selectivity of amiR136-12A, 10A, 12G and 12G/15U constructs were tested by luciferase assays. HEK293T cells were co-transfected with 50 ng of Luc reporters (containing exon 1 of the HTT gene with 16 CAG repeats, 57 CAG repeats or 85 CAG repeats) and 5, 10, 50, 150, or 500 ng of amiRNA constructs. The obtained results indicate a significant selectivity of the tested amiRNAs towards the mutated Htt proteins (FIG. 3A-3B).
Vector-based CAG-targeting interfering RNAs Lead to Allele-Selective Silencing of Mutant Proteins Huntingtin, Ataxin-3 and Atrophin-1.
In order to test the efficiency of shRNA-type interference RNA forming two mismatches with CAG sequence of interest, lentiviral vectors encoding sh12A, sh10A, sh12G molecules were constructed (containing SEQ ID NO.1-3 sequences from the Table 1). Human fibroblasts derived from patients with Huntington disease (GM04281) were transduced using lentiviruses at MOI of 10 and analyzed 7 days after transduction. Isolated protein was further analyzed using Western Blotting and silencing level of mutant and normal variant was assessed in comparison with reference protein (plectin) and negative control (scramble). Three analyzed reagents lead to allele-selective silencing of mutant huntingtin, leaving non-mutant protein on normal level. FIG. 4 shows CAG repeat-targeting interfering RNA in a form of shRNA decrease mutant huntingtin level in fibroblasts from HD patient (GM04281). Vector-based shRNA delivered as lentiviral vectors at MOI of 10. SCR - scramble control, N - normal huntingtin, M - mutant huntingtin; interfering RNAs with 2 mismatches: 10A, 12A, 12G were compared to interfering RNA A2 which contains one mismatch with a target sequence. The silencing efficiency and allele-selectivity of analyzed interfering RNAs was similar or even better than that of shA2 molecule containing only one mismatch with CAG sequence of interest.

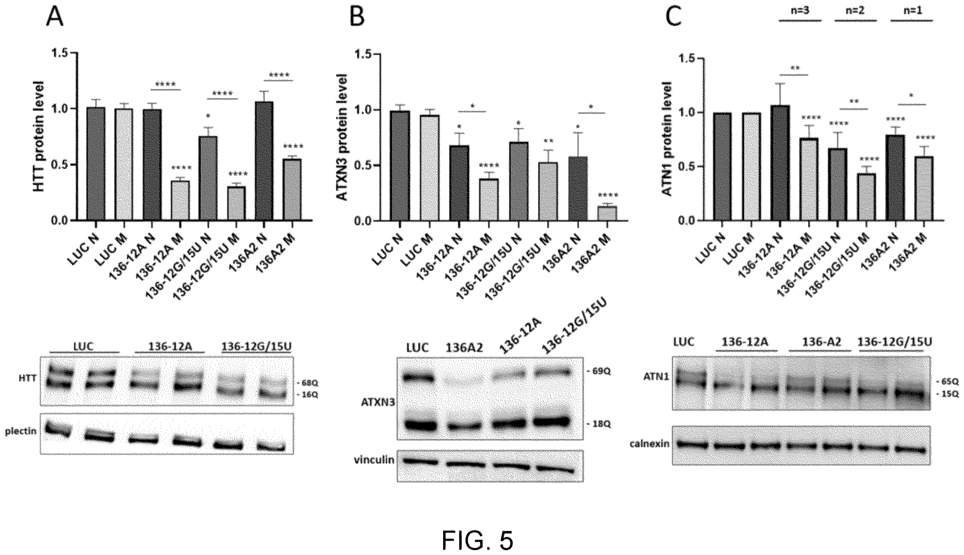

In order to test the efficiency of amiRNA-type interfering RNAs forming two or three mismatches with CAG sequence, lentiviral vectors encoding amiR136-12A and 12G/15U were constructed (containing SEQ ID NO.3 and 4 sequences from the Table 1). Human fibroblasts derived from patients with Huntington's disease were transduced and analysed as above. Silencing efficiency of the same amiRNAs was also tested on different model of polyglutamine diseases-SCA3 and DRPLA. FIG. 5 shows CAG repeat-targeting interfering RNA in a form of amiRNA decrease mutant huntingtin (A), ataxin-3 (B) and atrophin-1 (C) level in fibroblasts from patients (sequentially: GM04281, GM03561 and GM013717). Vector-based amiRNA delivered as lentiviral vectors at MOI of 10. SCR - scramble control, N - normal protein, M - mutant protein; interfering RNAs with 2 and 3 mismatches: 12A, 12G/15U were compared to interfering RNAA2 which contains one mismatch with a target sequence. n - number of biological replicates (if not marked, there are three replicates). Analyzed molecules lead to preferential silencing of mutant huntingtin, ataxin-3 and atrophin-1.

### Example 3: Mouse In Vivo Experiments

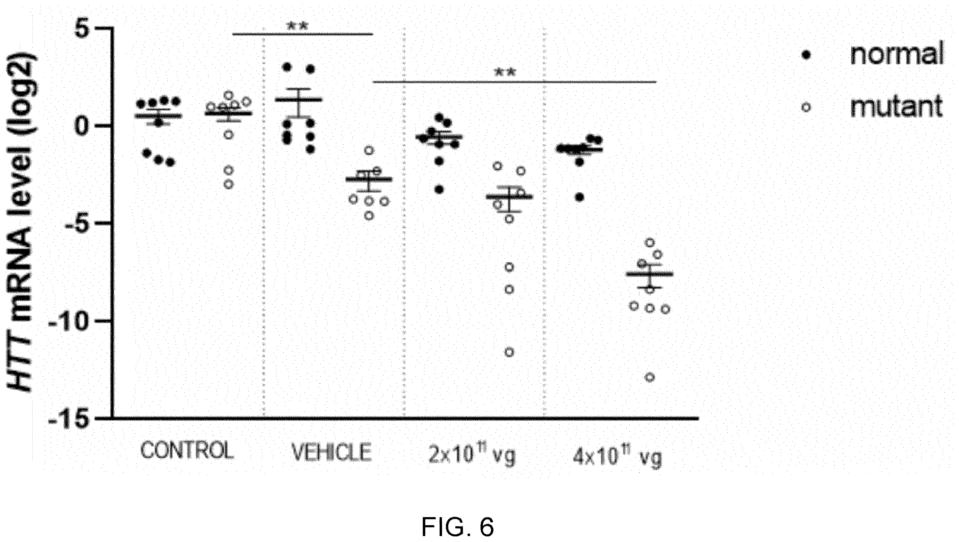

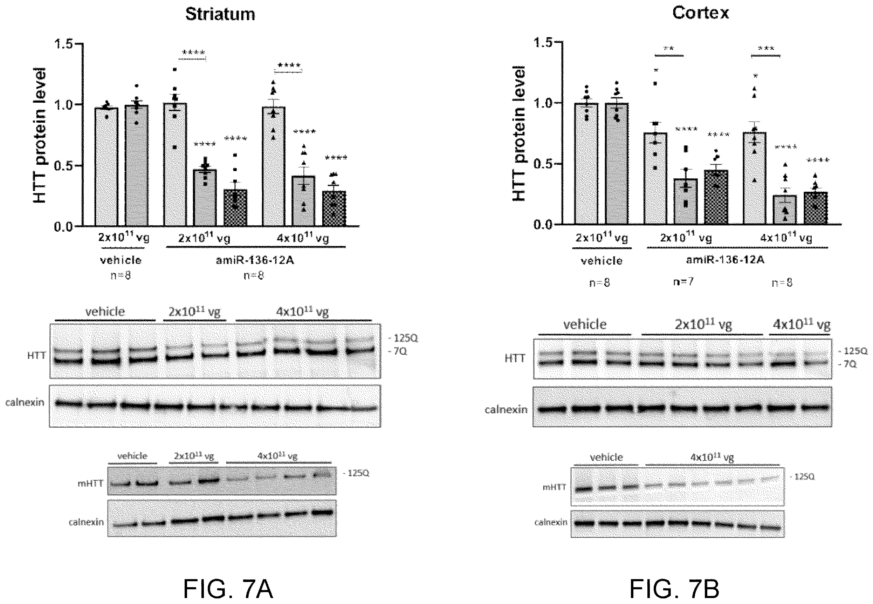

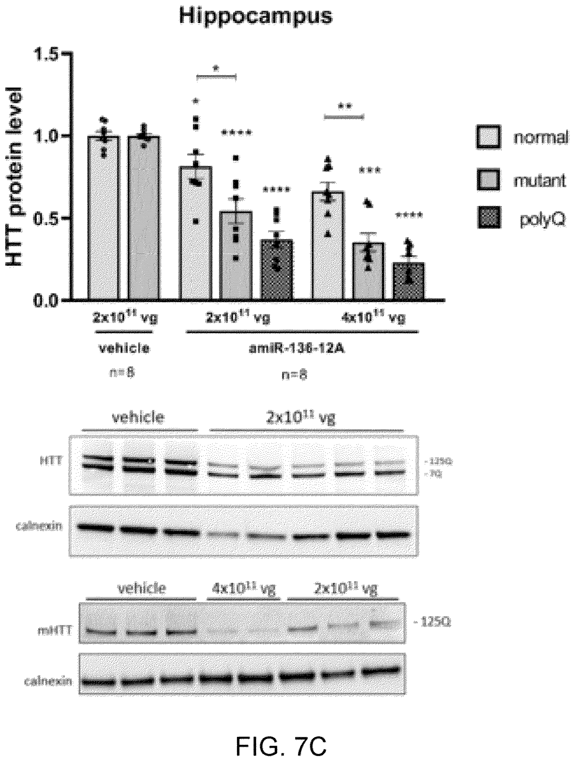

amiR136-12A construct was generated in AAV5 vector for direct delivery to the striatum of mice. amiRNA was expressed under the control of the Pol II promoter (a CAG promoter consisting of the cytomegalovirus immediate-early enhancer fused to the chicken β-actin promoter). Transgenic YAC128 mice, which express full-length human HTT with 125 CAG repeats interrupted by 9 CAA repeats, were used as the HD model. The presence of mouse Htt (mHtt) with 7 CAG repeats allows indirect analysis of allele selectivity in this model. To evaluate the efficacy of HTT silencing adult mice (12-weeks old) received bilateral intrastriatal injections of AAV5 carrying amiR136-12A at low and high doses (n=5 mice). One month post injection, the mice were sacrificed, and DNA, protein and RNA were isolated from the striatum, hippocampus and cortex for analysis of vector genome copies, analysis of protein levels by WB (FIG. 7A-7C) and analysis of transcript levels by RT-qPCR (FIG. 6), respectively. We observed allele-selective and significant reductions in mutant huntingtin levels in all of the brain regions analyzed (>50%).The reduction in HTT protein levels was dose-dependent and was observed for both doses of amiRNA. Western Blot analysis with polyQ antibody (detects only mutant HTT) confirmed our results.

FIG. 6 shows analysis of the HTT transcript level one month post injection of amiR136-12A in YAC128 mouse model. AAV5 vector was injected into the striatum in two doses 2×10^11/brain (low) and 4×10^11/brain (high). Control - untreated animals, vehicle control. Empty dots represent mutant HTT transcript level in each hemisphere, black dots represent normal, mouse Htt level.

FIGS. 7A-7C show Western blot analysis of HTT protein levels in the brains of YAC128 mice one month post injection of AAV5 vector in two doses. Each mark represents one hemisphere. Two antibodies were used to visualize HTT one detecting both forms of protein, mutant and normal, and the second detecting only mutant protein (polyQ antibody). Signal intensities of the protein bands were normalized to those of calnexin and compared using Student's t-test. The bars on the graph indicate the mean protein levels ± SEMs. P values are indicated by asterisks: *p<0.03, **p<0.01, ****p<0.0001. mHTT - mutant huntingtin, n - number of hemispheres.

### Example 4: Sequence Specificity

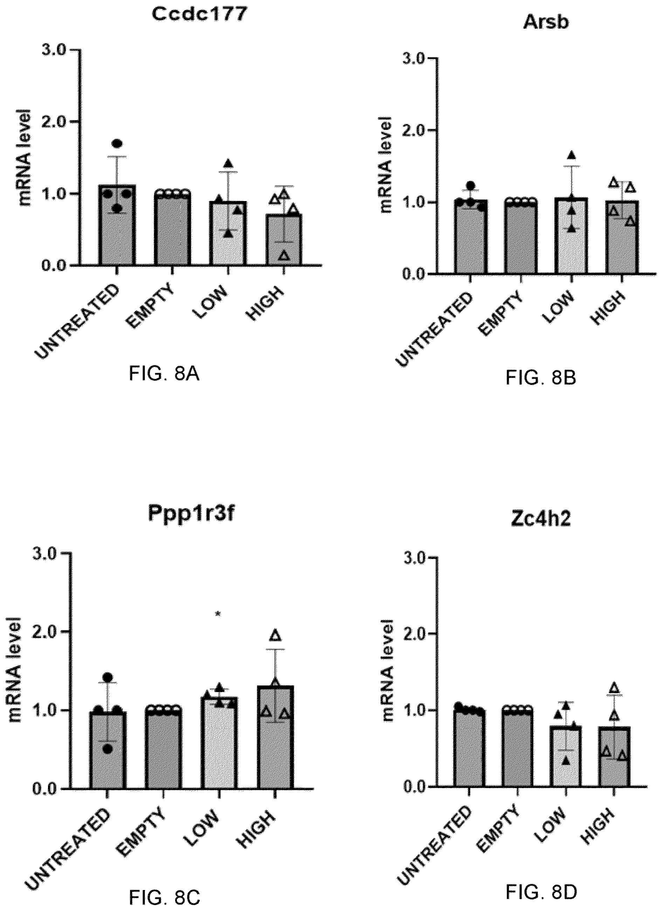

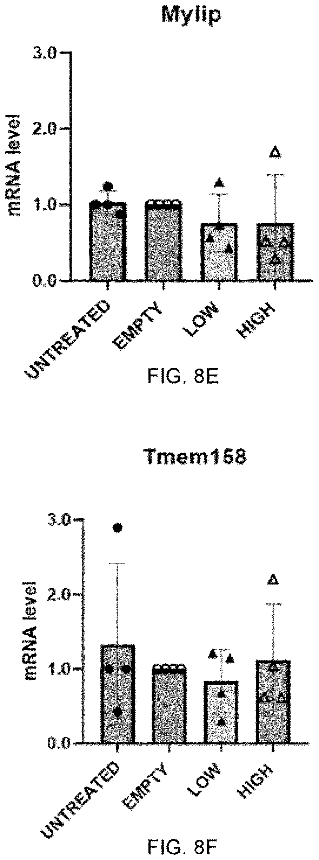

FIGS. 8A-8F show the analysis of transcript levels of genes with high level of complementarity to amiR136-12A (off-targets). To identify potential off-target sequences, the 12A sequence was mapped to the mouse genome using NCBI BLAST. The mouse genome does not contain a sequence fully complementary to the 12A sequence. Six off-target genes with 1 mismatch to 12A were selected for analysis (Mylip, Tmem158, Ppp1r3f, Zc4h2, Arsb, Ccdc177). All potential off-target sequences are located in the exons of these genes. AAV5 vector was injected into the striatum of the YAC128 mouse model at two doses of 2×10^11/brain (low) and 4×10^11/brain (high). RNA was isolated from the striatum using the Chomczynski method (phenolchloroform). Brain tissue for RNA isolation was collected from untreated controls, mice treated with control vector (empty) and amiR136-A2 at two doses (n=4). RT-qPCR analysis showed slight (not statistically significant) or no gene silencing with potential off-target sequences. Most importantly, the orthologous human Ccdc177 gene was not significantly silenced. The data obtained indicate that the amiR136-12A molecule does not significantly affect the expression of genes with potential complementary sequences.

While the invention has been described with respect to a limited number of embodiments, it will be appreciated that many variations, modifications and other applications of the invention may be made. Therefore, the claimed invention as recited in the claims that follow is not limited to the embodiments described herein.

**Table 1. Sequence list**

| Seq ID No. | Name | 5'-3'Guide strand sequence | Type of mismatches with sequence of interest |
|---|---|---|---|
| 1 | 10A | GCUGCUGC**A**GC**A**GCUGCUGCU | A:A; A:A |
| 2 | 12G | GCUGCUGC**A**GCU**G**GUGCUGCU | A:A; G:G |
| 3 | 12A | GCUGCUGC**A**GCUG**A**UGCUGCU | A:A; A:G |
| 4 | 12G/15U | GCUGCUGC**A**GCUG**G**UG**U**UGCU | A:A; G:G; U:G |

## Claims

1. A nucleic acid molecule composed of a duplex and loop, in which one of the duplex strands, the guide strand, comprises a sequence chosen from SEQ ID NO. 1-4, and the other strand of the duplex, the passenger strand, is at least 80% complementary to the guide strand, wherein the nucleic acid molecule forms a hairpin structure in a cell.

2. The molecule according to claim 1, **characterized in that** the duplex region is 19-30 base pairs long, and wherein preferably the first and the second strand of the duplex are connected by a loop 4 to 15 nt long, and further preferably wherein the molecule comprises modified CUG-type repeats in the guide strand having 1, 2, 3, or 4 substitutions causing formation of non-canonical base pairs by interaction with targeted CAG sequences in transcripts.

3. The molecule according to claim 1, wherein the molecule comprises 5' and 3' flanking sequences derived from natural miRNA, wherein precursor flanking sequences of natural length are shortened or not, and wherein preferably the molecule comprises a loop sequence derived from a natural miRNA or wherein preferably heterologous miRNA backbone sequence is a miR-136 backbone sequence.

4. An expression cassette comprising a regulated or constitutive promoter, wherein the promoter is functionally connected to a sequence encoding the nucleic acid molecule of claim 1.

5. The expression cassette according to claim 4, wherein the promoter is a polII or polIII RNA promoter.

6. An expression vector, comprising the expression cassette according to claim 4.

7. The expression vector according to claim 6, wherein the expression vector is a recombinant AAV (rAAV) and wherein preferably the capsid protein is an AAV5 capsid protein.

8. A eukaryotic host cell, comprising the nucleic acid molecule of claim 1.

9. A cell comprising the expression cassette of claim 8.

10. A eukaryotic host cell, comprising the expression vector of claim 6.

11. A method for inhibiting expression of a mutant CAG expanded gene allele in a cell, the method comprising delivering of a nucleic acid molecule of claim 1 to a eukaryotic host.

12. The method of claim 11, comprising introducing the nucleic acid molecule into the cell by expressing the expression cassette of claim 4 and wherein the method preferably comprises introducing the expression cassette into the cell by the expression vector of claim 6.

13. The method of claim 11, wherein the mutant CAG expanded gene allele is an allele of a gene that causes Huntington disease, spinocerebellar ataxia type 3 or dentatorubral pallidoluysian atrophy.

14. The method of claim 13, wherein the cell is cultured in vitro.

15. A method for treating a neurological disease caused by a mutant CAG expanded gene allele in a subject in need thereof, the method comprising inhibiting expression of a mutant CAG expanded gene allele in a cell of the subject by the method of claim 11, wherein preferably the mutant CAG expanded gene allele is an allele of a gene that causes Huntington disease, spinocerebellar ataxia type 3 or dentatorubral pallidoluysian atrophy.
